# EUROPEAN PATENT SPECIFICATION

(11) **EP 4 349 310 B1**
(45) Date of publication and mention of the grant of the patent: **08.07.2026**
(21) Application number: 23200897.9
(22) Date of filing: 29.09.2023
(51) Int. Cl.: A61F 9/009, A61F 9/008

(54) **OPHTHALMOLOGICAL LASER TREATMENT SYSTEM FOR TREATMENT OF TISSUE OF AN EYE OF A PERSON**
OPHTHALMOLOGISCHES LASERBEHANDLUNGSSYSTEM ZUR BEHANDLUNG VON GEWEBE EINES AUGES EINER PERSON
SYSTÈME DE TRAITEMENT LASER OPHTALMOLOGIQUE POUR LE TRAITEMENT DU TISSU DE L' OEIL D'UNE PERSONNE

(30) Priority: 04.10.2022 CH 11562022
(43) Date of publication of application: 10.04.2024
(73) Proprietor: Ziemer Ophthalmic Systems AG, 2562 Port (CH)
(72) Inventor: BERNAU, Werner, 3098 Köniz (CH); MÜLLER, Fabian, 2502 Biel (CH); RATHJEN, Christian, 28197 Bremen (DE); STEINLECHNER, Michael, 8006 Zürich (CH); STUDER, Thomas, 3286 Muntelier (CH)
(74) Representative: Rentsch Partner AG

(56) References cited:
- EP-A1- 3 323 393
- EP-A2- 1 306 068
- WO-A1-2021/119626
- DE-A1- 102014 225 635
- US-A1- 2017 290 703
- US-A1- 2020 330 268

## Description

### FIELD OF THE DISCLOSURE

The present disclosure relates to an ophthalmological laser treatment system for treatment of tissue of an eye of a person. In particular, the present disclosure relates to an ophthalmological laser treatment system for treatment of tissue of an eye of a person, wherein the ophthalmological laser treatment system comprises an ophthalmological laser treatment device and an ophthalmological patient interface.

### BACKGROUND OF THE DISCLOSURE

Ophthalmological treatment devices, which use a laser for eye treatment, are known. The ophthalmological treatment device has a laser source, which produces a pulsed laser beam. Additionally, the wavelength of the laser light produced by the ophthalmological treatment device is dependent on the type of eye treatment and is typically in the ultraviolet (190nm to 230nm) or infrared (780nm to 1100nm) range.

The laser beam is typically produced by a laser source arranged in a base station. The laser beam is then guided along a beam path to an application head, where the laser beam is focused onto a patient's eye.

The application head can be movably connected to the base device, for example by way of an articulated arm, wherein the articulated arm may simultaneously serve for optical beam guidance from the laser light source to the application head. Moreover, there are devices in which the application head is integrated into the base instrument or in which other device arrangements are provided.

Mechanical and optical coupling of the application head to the patient eye, for example to the cornea of the patient eye, is carried out by way of a patient interface, wherein the patient interface may comprise a transparent contact body, through which the laser pulses emerging from the projection lens are guided and which, by way of the mechanical contact with the cornea, fixes the latter with respect to the patient interface and the projection lens.

As an alternative to coupling by means of a contact body, provision can be made of liquid coupling, wherein a coupling liquid, for example a physiological saline solution, is situated between the cornea and the projection lens. The patient interface can be coupled to the patient eye by means of a negative-pressure cavity of the patient interface. The negative-pressure cavity is conventionally realized by a suction ring that is placed onto the cornea. Most suction rings have two sealing lips, which are attached to the cornea. Furthermore, there are variants which only have one ring and which generate a vacuum / negative pressure over the whole eye. In the known systems, the patient interface is coupled to the application head by means of e.g. a screw-in connection, bayonet closures or vacuum couplings.

The outer surface of the human eye, in particular the cornea and the area around the cornea are approximatively rotationally symmetric with respect to the optical axis of the eye. The conventional patient interfaces and in particular the eye contact surface of the conventional patient interfaces are therefore also rotationally symmetric such that the patient interface can be arranged coaxially on the cornea of the eye with respect to the optical axis of the eye for the desired rigid docking of the patient interface on the eye. At least one axis of the patient interface is thereby arranged coaxially with respect to the optical axis of the eye. Conventionally it is of high importance to position the patient interface coaxially with respect to the optical axis of the eye for determining the right position of the cutting pattern for the desired treatment of the eye. Nevertheless, the conventional positioning the patient interface limits the possible treatment surface of the eye for the treatment laser beam. Due to the design of the conventional patient interfaces it is almost not possible to treat portions of the eye, which are not arranged within a few millimeters of the optical axis of the eye. To increase the effective area, mirror elements are known, which are arranged in or next to the patient interface to deflect the laser beam and to direct the laser beam to the desired position. Nevertheless, the mirror elements do not increase the treatment surface; they only change the angle of incidence of the treatment laser beam.

The use cases for the conventional ophthalmological laser treatment device are therefore limited to treatments of the immediate vicinity of the optical axis of the eye, in particular to treatments of specific parts of the cornea of the eye. Typical use cases are a refractive surgery, like a LASIK surgery, or a cataract surgery. With a conventional ophthalmological laser treatment device it is currently not possible to perform surgeries or treatment of other parts of the human eye, which are not located in the immediate vicinity of the optical axis of the eye.

The document WO 2021/119626 A1 relates to a systems and methods for performing laser operations on the structures of the eye, including the drain angle, trabecular mesh and the area of the eye where the iris and cornea meet.

The document DE 10 2014 225 635 A1 relates to a device and a method for fixing the relative geometric position of an eye to an ophthalmological diagnosis and/or therapy system, as well as an ophthalmological diagnosis and/or therapy system with such a device. A device comprising a container having an upper edge designed to establish a mechanically strong connection between the container and the ophthalmological diagnosis and/or therapy system, and a lower edge that can be applied to the eye in a form-fitting manner, and suction means comprising a suction lip along the lower edge with a plurality of suction structures, a suction opening in a casing area of the container for connecting a suction device and being in direct connection with all suction structures, in direct communication, wherein at least two suction structures are at a first distance from a central axis of the device and at least two suction structures are at a second distance from the central axis of the device.

### SUMMARY OF THE DISCLOSURE

It is an object of the present disclosure to provide an ophthalmological laser treatment system for treatment of tissue of an eye of a person. In particular, it is an object of the present disclosure to provide an ophthalmological laser treatment system for treatment of tissue of an eye, which does not have at least some of the disadvantages of the prior art, in particular, which enable to treat areas of the eye, which are not located in the immediate vicinity of the optical axis of the eye.

According to the present disclosure, these objects are addressed by the features of the independent claim. In addition, further advantageous embodiments follow from the dependent claims and the description.

According to the present disclosure, an ophthalmological laser treatment system for treatment of tissue of an eye of a person is specified. The ophthalmological laser treatment system comprises an ophthalmological laser treatment device. The ophthalmological laser treatment device comprising a base station having a treatment laser source configured to generate a treatment laser beam, an arm and an application head, wherein the arm is configured to guide the treatment laser beam from the base station to the application head. The ophthalmological laser treatment system further comprises an ophthalmological patient interface, which comprises a coupling portion, configured to be arranged on the application head, and an eye fixation portion, configured to be arranged on the eye. The eye fixation portion comprises a cornea contacting element, configured to contact at least partially the cornea of the eye, and a sclera contacting element, configured to contact at least partially the sclera of the eye, for firmly positioning the ophthalmological patient interface on the eye, wherein the cornea contacting element and the sclera contacting element define/determine a rotationally asymmetric contact surface of the eye fixation portion configured to contact the eye. In other words, the contact surface of the eye fixation portion, which is configured to contact the sclera and the cornea of thee eye is rotationally asymmetric. The ophthalmological patient interface further comprises a through opening, extending through the coupling portion and the eye fixation portion, wherein the through opening is configured to enable the treatment laser beam from the application head to pass through the ophthalmological patient interface to penetrate a target volume of tissue of the eye.

The ophthalmological patient interface is configured to be attached on the sclera and the cornea such that the through opening is configured to be arranged on areas of the eye, which are located relatively far away from the optical axis of the eye, like the sclera of the eye. The ophthalmological laser treatment system comprising the specific ophthalmological patient interface according to the present disclosure enables therefore to treat for example diseases of the sclera with the treatment laser beam through the through opening with the required precision, which were previously, with conventional ophthalmological laser treatment devices, not reachable. The ophthalmological laser treatment system enables to treat for example a pterygium arranged on the sclera of the eye using the treatment laser beam, which is guided through the ophthalmological patient interface, which is rigidly positioned above the target tissue comprising the pterygium. The treatment of the pterygium may comprise to cut, by laser treatment, donor tissue (from the other eye) and "glue" it on the place of the other eye where the pterygium was. Gluing may be performed by using a specific glue, which is applied at least partially between the donor tissue and the treatment surface, were the pterygium was. The glue is for example a fibrin adhesive. The removal of the pterygium tissue is for example performed via laser treatment.

Penetration of the target volume of tissue of the eye comprises for example to apply/direct the treatment laser beam on a surface of the eye, for example on the outer surface of the sclera, or to apply / direct the treatment laser beam into interior portions of the eye, for example in the cornea or the lens of the eye. Penetration further includes to form or to "drill" holes in the eye of the person and to form cuts in the eye, to cut parts of the eye of the person, via the treatment laser beam. The rotationally asymmetric contact surface provides the possibility to apply the ophthalmological patient interface non-coaxially with the optical axis of the eye. The ophthalmological patient interface according to the present disclosure can be placed laterally next to the optical axis of the eye, which substantially increases the possible treatment surface for the treatment laser beam.

Rotational symmetry, also known as radial symmetry, in geometry, is the property a shape has when it looks the same after some rotation by a partial turn. Rotational asymmetry on the contrary is, according to the present disclosure, the property a shape has when it looks not the same after some rotation by a partial turn. Certain geometric objects are partially symmetrical, but still rotational asymmetric, when rotated at certain angles such as squares rotated 90°, however the only geometric objects that are truly rotationally symmetric at any angle are spheres, circles, spheroids and solids of revolution.

The optical axis of the eye is for example the straight line between the centers of curvature of refractive surfaces of the eye. The optical axis of the eye may further be the visual axis of the eye, which runs from the fovea centralis of the eye through the nodal point of the eye to the object of fixation. The optical axis of the eye may further be the line of sight of the eye, which is the straight line between the pivot point of the eye and the fixation object. The optical axis of the eye may further be the pupil axis, which is the straight line between the center of the cornea and the center of the pupil. Angular deviations of this axis are usually less than 5 degrees. The optical axis may further be a geometrical combination (best fit) between two or more of the above-mentioned different axis of the eye.

In an embodiment, the ophthalmological laser treatment system comprises an optical mirror element arranged between the application head and the ophthalmological patient interface or within the ophthalmological patient interface, wherein the optical mirror element is configured to deflect the treatment laser beam incidenting from the application head at a specific angle, wherein the angle of incidence of the treatment laser beam on the eye with respect to an optical axis of the eye is determined by the inclined position of the ophthalmological patient interface with respect to the optical axis on the eye and the specific angle of the optical mirror element. In other words, the optical mirror element is configured to deflect the treatment laser beam coming from the application head, which increases the effective area or the possible treatment surface of the treatment laser beam onto or into the eye. The ophthalmological patient interface is not configured to be arranged on the eye co-axially or co-linear with respect to the optical axis of the eye. The angle of incidence of the treatment laser beam onto or into the eye is therefore determined by the inclined position of the ophthalmological patient interface with respect to the optical axis of the eye, when applied on the eye. In case, the ophthalmological laser treatment system comprises the optical mirror element, the angle of incidence of the treatment laser beam is further determined by the specific angle of the optical mirror element. The specific angle is the angle between a mirror surface of the optical mirror element and the incidenting treatment laser beam.

In an embodiment, the optical mirror element comprises at least one adjustable mirror, which is configured to adjust the specific angle based on a position of the tissue of the eye to be treated and the position of the ophthalmological patient interface on the sclera and cornea of the eye to adjust the angle of incidence of the treatment laser beam on the eye. The optical mirror element comprises according to this embodiment for example small electrical engines, which are configured to adjust the orientation of the mirror surface with respect to the incidenting treatment laser beam, whereby the angle of incidence of the treatment laser beam can be adjusted to optimize the treatment of the target volume by the treatment laser beam. In another embodiment, the optical mirror element may comprise a mechanical system operable by a person manually to adjust the orientation of the mirror surface.

In an embodiment, the ophthalmological laser treatment system further comprises an optical imaging device configured to generate optical image data of the eye of the person, which are configured to be used for positioning of the ophthalmological patient interface on the sclera and cornea of the eye and / or for adapting of the position of the ophthalmological patient interface on the sclera and cornea of the eye. The optical imaging device is for example arranged in the beam path between the application head and the ophthalmological patient interface, such that the optical imaging device is configured to capture images in the same direction of the treatment laser beam from the application head towards the eye. In other words, the optical imaging device provides image data of the target surface of the treatment laser beam of the eye, when connected to the eye. For example, the ophthalmological patient interface is placed on the sclera and the cornea of the eye laterally next to the optical axis of the eye. In a next step, the application head is arranged on the coupling portion of the ophthalmological patient interface. The optical imaging device provides optical image date of the eye of the person, in particular of the treatment surface arranged below of the through opening of the ophthalmological patient interface. Using the optical image data, the position of the ophthalmological patient interface might be adapted such that the tissue of the eye to be treated is advantageously reachable. The adapting process might be performed automatically or manually. In a further embodiment, the treatment laser beam is controlled, based on the optical image data, such that the desired treatment of the tissue is realized.

In a further embodiment, the ophthalmological laser treatment system comprise the optical mirror element and the optical imaging device, wherein the optical imaging device is configured to provide image data of the target volume of the eye, which is reachable by the treatment laser beam deflected by the optical mirror element. In other words, the optical imaging device is configured to provide image data deflected by the mirror surface of the optical mirror element. The optical imaging device is positioned such in the beam path that light from the treatment surface, is deflected by the mirror element reaches the optical imaging device. This advantageously increases the positioning accuracy of the ophthalmological patient interface, which increases the quality of the treatment by the ophthalmological laser treatment system.

In a further embodiment, the optical imaging device further comprises an optical depth module, which is configured to provide depth information of the eye, which are configured to be used for positioning of the ophthalmological patient interface on the sclera and cornea of the eye and / or for adapting of the position of the ophthalmological patient interface on the sclera and cornea of the eye. The treatment of the eye by the ophthalmological laser treatment system often includes a 3D treatment, for example, to treat not only a surface of the sclera but to treat a volume of the sclera. It is therefore of importance to know a depth of the treatment volume, for example to know the depth extension of a pterygium and the surface extension of the pterygium, in other words, to know the exact 3D extension of the pterygium. This enables to optimize the treatment by the ophthalmological laser treatment system.

In an embodiment, the optical depth module, which is configured to provide depth information of the eye, is an imaging device based on the Scheimpflug principle, or an optical coherence tomography device, a confocal microscopy device, a device for structured lighting and / or a device for stereoscopic vision.

In a further embodiment, the optical image data obtained by the optical imaging device and the depth information / data are in combination displayed and / or used for positioning of the ophthalmological patient interface on the sclera and cornea of the eye and / or for adapting of the position of the ophthalmological patient interface on the sclera and cornea of the eye. Further, the optical image data obtained by the optical imaging device and the depth information / data are in combination used to control the treatment laser beam for the treatment of the eye tissue.

In a further embodiment, the ophthalmological laser treatment system comprises a control module. The control module is for example arranged in the base station. The control module is configured to determine, by a processing unit of the control module, the target volume of tissue to be treated using the captured image data and / or the depth information, of the optical imaging device. The control module is further configured to control, by the processing unit, the treatment laser beam for penetration of the target volume, using the determined target volume of tissue to be treated. The control module controls for example a laser scanner to penetrate the desired target volume of the eye.

In an embodiment, the contact surface of the eye fixation portion of the ophthalmological patient interface has a circular outer contour, and the rotational asymmetry of the contact surface is determined by the arrangement of the cornea contacting element and the sclera contacting element within the circular outer contour. In this embodiment, the rotational asymmetry is determined for example by the shape of the cornea contacting element and the shape of the sclera contacting element. For example, the cornea contacting element has a quarter ring shape having a first thickness and the sclera contacting element has a three-quarter ring shape having a second thickness, which differs from the first, thereby forming the rotational asymmetric contact surface.

In an embodiment, the cornea contacting element of the ophthalmological patient interface comprises a rotationally asymmetric suction opening configured to be positioned at least partially, preferably entirely, on the cornea of the eye and configured to be fluidically connected to a negative pressure, and / or wherein the sclera contacting element of the ophthalmological patient interface comprises a rotationally asymmetric suction opening configured to be positioned at least partially, preferably entirely, on the sclera of the eye and configured to be fluidically connected to a negative pressure for positioning the ophthalmological patient interface on the eye. In this embodiment, the rotational asymmetry is determined for example by the asymmetric suction opening of the cornea contacting element, which contacts the cornea of the eye and / or for example by the asymmetric suction opening of the sclera contacting element, which contacts the sclera of the eye, when applied on the eye.

In an embodiment, the negative pressure applied to the suction opening of the cornea contacting element is higher than the negative pressure applied to the suction opening of the sclera contacting element. The sclera tissue has different biomechanical properties compared to the cornea tissue, which results in different optimal negative pressures for coupling without harming the different eye tissues. Having different negative pressures for the different kind of suction openings creates the possibility to apply for the cornea and the sclera different pressures to achieve the desired suction effect in combination with eye protection.

In an embodiment, the suction openings of the cornea contacting element has a partial-ring-shape. In a further embodiment, the suction opening of the sclera contacting element has a partial-ring-shape. A partial ring or a sub-ring is a partition of a ring, for example a quarter, a third or two thirds of a ring.

In an embodiment, the suction opening of the cornea contacting element is configured to be positioned entirely on the cornea of the eye, when applied on the eye. According to this embodiment, the suction opening of the cornea contacting element has preferably a shape which, corresponds to the shape of the cornea, in particular which corresponds to the shape of the specific area of the cornea.

In an embodiment, the suction opening of the sclera contacting element is configured to be positioned entirely on the sclera of the eye, when applied on the eye. According to this embodiment, the suction opening of the sclera contacting element has preferably a shape, which corresponds to the shape of the sclera, in particular which corresponds to the shape of an area of the sclera onto which the sclera contacting element is applicable.

In a further embodiment, the suction opening of the cornea contacting element and / or the suction opening of the sclera contacting element extend on the cornea and the sclera respectively. According to this embodiment, the suction opening of the cornea contacting element is configured to be arranged on the cornea and the sclera, preferably for the most part on the cornea, and the suction opening of the sclera contacting element is configured to be arranged on the sclera and the cornea, preferably for the most part on the sclera.

In an embodiment, the through opening is configured to be at least partially filled with a coupling liquid, wherein the cornea contacting element and/or the sclera contacting element comprises a sealing or a plurality of seals or sealings. Wherein the sealing is configured to seal at least partially between the surface of the eye and the ophthalmological patient interface when the ophthalmological patient interface is applied on the eye. In an embodiment, the sealing comprises a sealing lip or a balloon seal or a sealing gel. The sealing is preferably arranged on the transition portion between the sclera and the cornea, preferably partially on the ring shaped contact surface, for example, between the suction opening of the cornea contacting element and the suction opening of the sclera contacting element. In a further embodiment, the sealing comprises a membrane, which is configured to absorb coupling liquid to provide the required sealing properties.

In an embodiment, the through opening functions as a reservoir for the coupling liquid. In a further embodiment, the coupling liquid is a physiological saline solution.

In an embodiment, the ophthalmological laser treatment system comprises a liquid dispenser, which is configured to dispense coupling liquid into the through opening. The liquid dispenser is for example configured to dispense the coupling liquid continuously or on demand or in dependence of the filling lever of the coupling liquid in the through opening. The ophthalmological patient interface comprises, for example, a filling possibility, for example a connection to the liquid dispenser. In a further embodiment, the filling possibility is an opening arranged laterally on the ophthalmological patient interface, for example an opening in the eye fixation portion and / or in the coupling portion, which is accessible for the liquid dispenser even when the ophthalmological patient interface is applied on the eye and when the application head is arranged on the coupling portion of the ophthalmological patient interface.

In an embodiment, the through opening defines an opening in the eye fixation portion, which is enclosed by the sealing and/or the suction opening(s).

In an embodiment, the eye fixation portion comprises a flat contact body arranged in in the through opening, wherein the contact body is at least partially transparent for the treatment laser beam to enable the penetration by the treatment laser beam of the target volume of tissue of the eye, wherein the flat contact body comprises a flat contact surface, which forms part of the rotationally asymmetric contact surface and which is configured to conform the sclera and / or the cornea of the eye. In other words, the flat contact surface deforms the sclera and / or the cornea of the eye, in case the flat contact body contacts the eye.

In an embodiment, the cornea contacting element comprises a form-fitted contact body comprising a form-fitted contact surface forming part of the rotationally asymmetric contact surface, wherein the form-fitted contact surface is form-fitted to a shape of the outer surface of the cornea of the eye, wherein the form-fitted contact surface is configured to be at least partially in form-fitting contact with the cornea of the eye for firmly positioning the ophthalmological patient interface on the eye. The surface tension of the tear film provides an advantageous docking of the form-fitted contact body on the eye, which advantageously improves the docking of the entire ophthalmological patient interface on the eye. For example, the form-fitted contact surface corresponds to an asphericity of the cornea of the eye. The form fitted contact body is for example made of glass.

The ophthalmological laser treatment system according to one of the preceding claims, wherein the cornea contacting element and / or the sclera contacting element comprises protrusions, which are configured to penetrate tissue of the sclera or the cornea respectively. The protrusions have for example a pyramid shape. In a further embodiment, the protrusions are arranged only partially along the cornea-contacting element and / or the sclera-contacting element. In a further embodiment, the protrusions are arranged only on the sclera-contacting element. The sclera tissue is relatively soft compared to the cornea tissue, which is advantageous for the required penetration of the spikes in the sclera tissue to position the ophthalmological patient interface rigidly on the eye.

In an embodiment, the sealing is arranged radially outside with respect to other parts or portions of the ophthalmological patient interface, in particular radially outside of the protrusions or the suction openings. In a further embodiment, the sealing is arranged ring shaped around the rotationally asymmetric contact surface. The sealing is advantageous reliable and simple in case the sealing is arranged uninterrupted, continuously around the rotationally asymmetric contact surface.

In an embodiment, the cornea contacting element and / or the sclera contacting element comprise at least one suction cup having a flexible contact body configured to contact the eye, thereby forming part of the rotationally asymmetric contact surface, for positioning the ophthalmological patient interface with respect to the eye. The suction cup is for example a round rubber part arranged laterally next to the eye fixation portion of the ophthalmological patient interface. The suction cup is configured to contact the eye such that the ophthalmological patient interface is arranged rigidly on the eye when applied on the eye.

In an embodiment, a bracket is arranged between the suction cup and the eye fixation portion, wherein the bracket extends from the eye fixation portion at a specific angle and at a specific length such that the suction cup is configured to contact the cornea of the eye in particular coaxially with respect to the optical axis of the eye.

In an embodiment, the sclera contacting element comprises at least one, preferably a plurality of, spoon-shaped gripper arm(s) extending from the coupling portion and / or the eye fixation portion, wherein the spoon-shaped gripper arm(s) extend arc-shaped, the arc having an eye-ball radius, from the eye fixation portion, thereby forming part of the rotationally asymmetric contact surface, for positioning the ophthalmological patient interface with respect to the eye. The spoon-shaped gripper arm(s) follow the shape of the eyeball, such that the surface tension of the tear film provides the desired rigid docking of the ophthalmological patient interface on the eye. In a further embodiment, at least one of the spoon-shaped gripper arms comprises, preferably at its tip area, a suction cup for firmly positioning the ophthalmological patient interface on the eye. In a further embodiment, at least one of the spoon-shaped gripper arms comprises at least one protrusion, which is configured to penetrate the surface of the eye for rigidly positioning / docking of the ophthalmological patient interface on the eye.

In an embodiment, the ophthalmological patient interface further comprises a safety coupling, which comprises a first part connected rigidly to the spoon-shaped gripper arm(s) and a second part connected rigidly to the eye fixation portion and/or the coupling portion, wherein the first part is connected detachable to the second part, such that the coupling opens when a force engaging on the ophthalmological patient interface reaches or surpasses a predefined threshold force.

In an embodiment, the first part and the second part of the safety coupling are connected detachable by means of magnets or by means of a vacuum or by means of a spring or by means of a detachable interlocking connection between the first part and the second part.

In an embodiment, the coupling portion and the eye fixation portion of the ophthalmological patient interface are at least two individual parts, which are configured to be coupled for forming at least partially the ophthalmological patient interface. The coupling portion and the eye fixation portion are for example coupled by a detachable interlocking connection

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- **Figure 1**: shows a perspective view illustrating schematically an ophthalmological laser treatment system;
- **Figure 2**: shows a block diagram illustrating schematically an ophthalmological laser treatment system with an ophthalmological patient interface;
- **Figure 3**: shows schematically different components of the ophthalmological laser treatment system including the ophthalmological patient interface;
- **Figure 4**: shows schematically a cross section of a first conventional ophthalmological patient interface applied on an eye;
- **Figure 5**: shows schematically a cross section of a second conventional ophthalmological patient interface applied on an eye;
- **Figure 6**: shows schematically a cross section of an ophthalmological patient interface according to a first embodiment;
- **Figure 7**: shows schematically a cross section of an ophthalmological patient interface according to a second embodiment;
- **Figure 8**: shows schematically a cross section of an ophthalmological patient interface according to a third embodiment;
- **Figure 9**: shows schematically a cross section of an ophthalmological patient interface according to a fourth embodiment;
- **Figure 10**: shows schematically a cross section of an ophthalmological patient interface according to a fifth embodiment;
- **Figure 11**: shows schematically a cross section of an ophthalmological patient interface according to a sixth embodiment;
- **Figure 12**: shows schematically a cross section of an ophthalmological patient interface according to a seventh embodiment;
- **Figure 13**: shows schematically a perspective view of an ophthalmological patient interface according to an eight embodiment;
- **Figure 14**: shows schematically a perspective view of an ophthalmological patient interface according to a ninth embodiment;
- **Figure 15**: shows schematically a top view of an ophthalmological patient interface according to a tenth embodiment;
- **Figure 16**: shows schematically a top view of an ophthalmological patient interface according to an eleventh embodiment;
- **Figure 17**: shows schematically a top view of an ophthalmological patient interface according to a twelfth embodiment;
- **Figure 18**: shows schematically a cross section of an ophthalmological patient interface according to a thirteenth embodiment;
- **Figure 19**: shows schematically a block diagram for controlling the ophthalmological laser treatment system by a control module.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

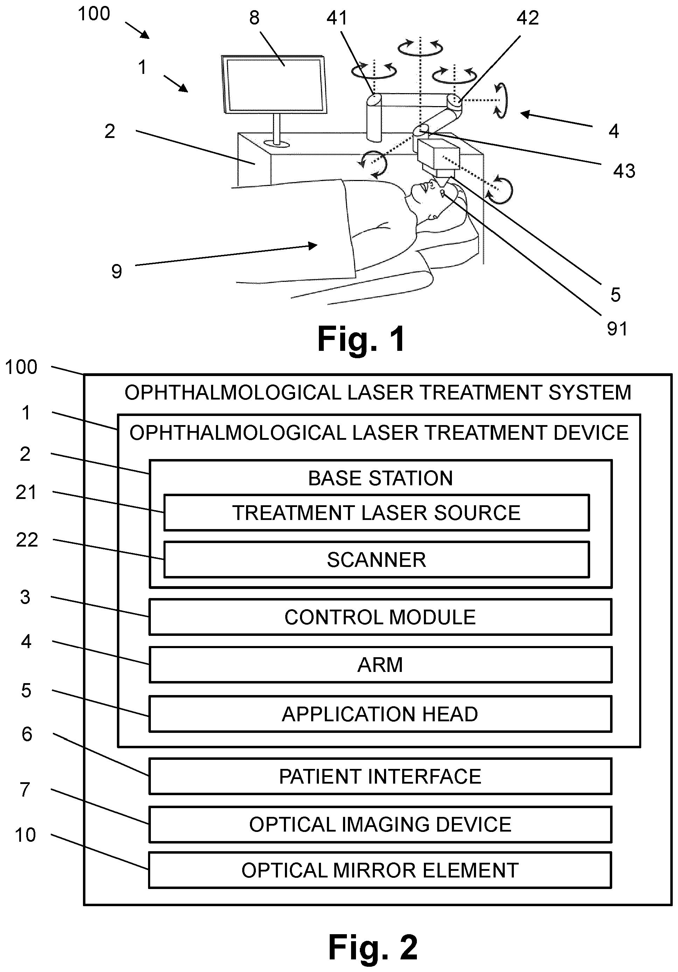

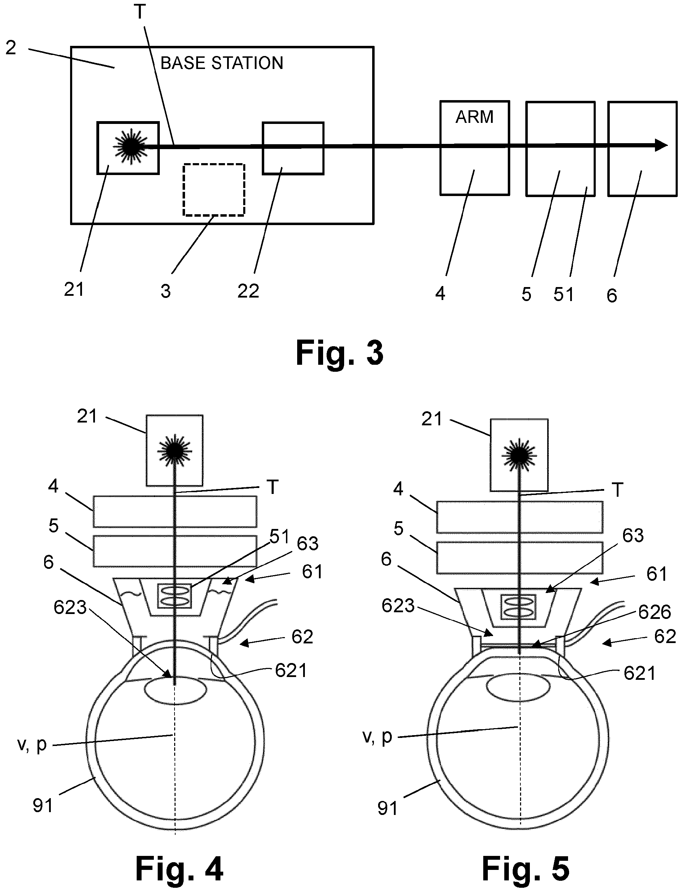

**Figure 1** shows a perspective view illustrating schematically an ophthalmological laser treatment system 100. **Figure 2** shows a block diagram illustrating schematically the ophthalmological laser treatment system 100 with an ophthalmological patient interface 6. **Figure 3** shows schematically different components of the ophthalmological laser treatment system 100.

The Figures 1 to 3, schematically illustrate modules and/or elements of various embodiments of the ophthalmological laser treatment system 100 and provide exemplary sequences or arrangement of modules and/or elements, including modules and/or elements in a beam path T. Some modules and/or elements shown in a particular Figure may be combined with modules and/or elements shown in another Figure.

The ophthalmological laser treatment system 100 comprises an ophthalmological laser treatment device 1 comprising a base station 2. The base station 2 is configured as a fixed or mobile apparatus. The ophthalmological laser treatment device 1 has a treatment laser source 21 arranged in the base station 2, which generates a treatment laser beam T. The base station 2 further includes, for example, a power supply and other auxiliary subsystems necessary for operation of the ophthalmological laser treatment device 1.

The treatment laser source 21 is configured, for example, to generate an ultraviolet or infrared treatment laser beam T having a wavelength of between 190 nm and 230 nm. For example, the treatment laser source 21 comprises an excimer or a solid-state laser, which produces such an ultraviolet treatment laser beam T. The excimer laser uses a combination of a noble gas and a reactive gas under high pressure and electrical stimulation to generate the treatment laser beam T. In particular, an excimer laser using argon as the noble gas and fluoride as the reaction gas may be used as the treatment laser source 21.

In an embodiment, the treatment laser beam T is a pulsed laser beam. In an embodiment, the treatment laser source 21 is configured to generate femtosecond laser pulses, which have pulse widths of typically from 10 fs to 1000 fs (1 fs = 10⁻¹⁵ s).

The base station 2 includes a scanner 22, which is configured to steer the treatment laser beam T delivered by the treatment laser source 21 onto or into treatment points on a treatment pattern (comprising a laser trajectory).

The ophthalmological laser treatment device 1 comprises an application head 5. The application head 5 is designed to guide the treatment laser beam T into or onto the eye 91 of a patient 9 (as shown, for example, in Figure 1). The application head 5, for this purpose, can comprise focusing optics 51 configured to focus the treatment laser beam T onto one or more treatment points inside or on the eye 91, in particular the cornea or the sclera for a pointwise tissue disruption or ablation. The focusing optics 51 comprise a lens system having one or more optical lenses. Depending on the embodiment, the focusing optics 51 comprise one or more movable or deformable lenses and/or a drive for moving the entire focusing optics in order to set and adjust the focal depth, or the treatment height, in the projection direction along the projection axis.

The ophthalmological laser treatment system 100 comprises an ophthalmological patient interface 6. The application head 5 is preferably fixed onto the eye 91 by means of the ophthalmological patient interface 6, which is coupled to the eye for example using negative pressure. Different embodiments of the ophthalmological patient interface 6 of the present disclosure will be described in more detail with reference to the Figures 4 to 17.

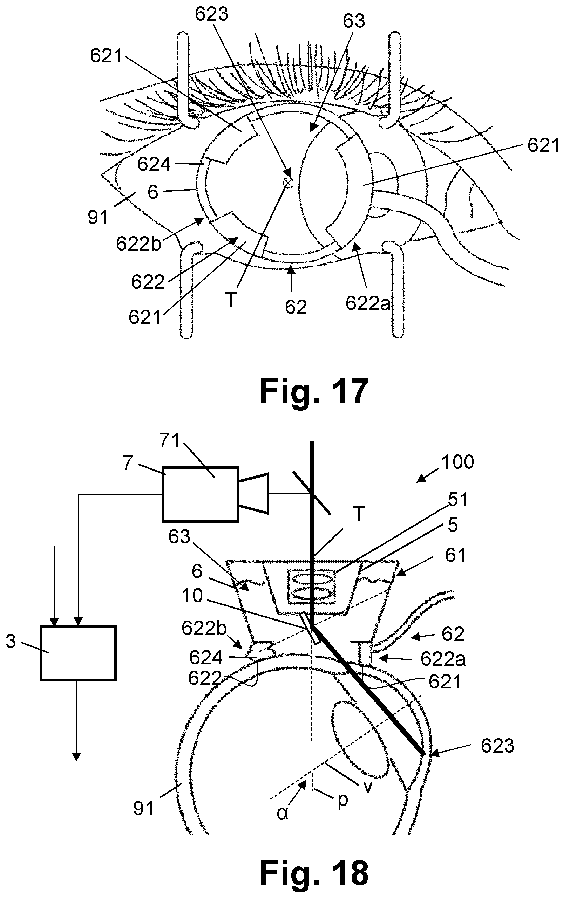

Depending on the embodiment, the ophthalmological laser treatment system 100 further comprises an optical imaging device 7 and an optical mirror element 10. The optical imaging device 7 and the optical mirror element 10 will be explained in more detail with reference to Figure 18 below.

The ophthalmological laser treatment device 1 comprises an arm 4 arranged between the base station 2 and the application head 5. The arm 4 is configured to provide a beam path for the treatment laser beam T, such that the treatment laser beam T is guided along the inside of the arm 4 from the base station 2 to the application head 5. In an embodiment, the arm 4 comprises one or more joints 41 (as shown in Figures 1) such that the application head 5 is movable and/or rotatable with respect to the base station 2. Each rotatable joint 41 comprises a mirror arranged in the beam path to reflect the treatment laser beam T along the arm 4.

The ophthalmological laser treatment system 100 is controlled by a control module 3, by controlling the treatment laser source 21 and the scanner 22, as well as by controlling additional modules of the ophthalmological laser treatment system 100 arranged in the beam path of the treatment laser beam T.

The ophthalmological laser treatment system 100 optionally includes a user interface comprising, for example, one or more user input devices, such as a keyboard, and one or more output devices, such as a display 8 (as shown in Figures 1). The display 8 may also be an input device. The user interface is configured to receive user inputs from an eye treatment professional, in particular based on, or in response to, information displayed to the eye treatment professional using the one or more output devices.

The **Figures 4** and **5** show schematically a cross section of a first and second conventional ophthalmological patient interface 6 applied on an eye 91 of a person 9. The Figures 4 and 5 show schematically the treatment laser source 21, the arm 4 and the application head 5 with the focusing optics 51. The Figures further show schematically the treatment laser beam T and its beam path from the treatment laser source 21 to the eye 91. The conventional ophthalmological patient interface 6 comprises a coupling portion 61, which is configured to be attached to the application head 5, and an eye fixation portion 62, which is configured to contact the cornea of the eye 91. The eye fixation portion 62 comprises a contact surface, which is configured to contact the eye 91, when the ophthalmological patient interface 6 is applied on the eye 91. The Figures further show schematically a ring shaped suction opening 621, which extends ring shaped along the entire contact surface of the eye fixation portion 62. The ring shaped suction opening is connected to a negative pressure or vacuum such that the eye fixation portion 62 is rigidly attachable to the eye 91. The Figures further show that the ophthalmological patient interface 6 comprises a through opening 63, extending through the coupling portion 61 and the eye fixation portion 62. The treatment laser beam T is guided along the through opening 63 to a treatment surface 623 on or in the cornea of the eye 91. The through opening 63 is configured to enable the treatment laser beam T from the application head 5 to pass through the ophthalmological patient interface 6 to penetrate a target volume of tissue of the eye 91. Figure 5 additionally comprises a contact body 626, which is arranged in the through opening 63 and which is configured to conform / deform the cornea of the eye 91, for example for a conventional LASIK surgery.

The Figures 4 and 5 further show the optical axis v of the eye 91 and an optical axis p of the ophthalmological patient interface 6. The trough opening 63 extends along the axis p. Both axis v, p are arranged coaxially with respect to each other. It is of high importance that these two axis are arranged coaxially with respect to each other for an optimal treatment result using the conventional ophthalmological patient interface 6 for the desired precise treatment. The coaxially alignment is achieved by a rotational symmetry of the contact surface of the eye fixation portion 62, which perfectly aligns with the rotational symmetric surface of the cornea of the eye 91.

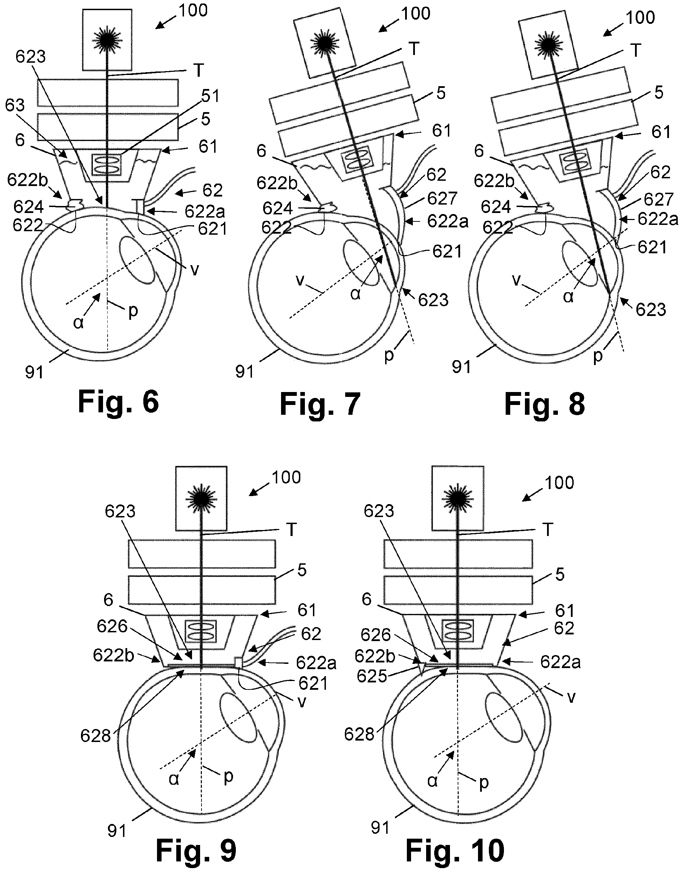

Reference is now made to the embodiments of the present disclosure as shown in the Figures 6 to 19.

The **Figures** 6 to **18** show at first sight that the ophthalmological patient interface 6 of the ophthalmological laser treatment system according to the present disclosure with its axis p is not configured to be arranged coaxially and is also not configured to be arranged co-linear with respect to the optical axis v of the eye 91, when applied on the eye. The axis p of the ophthalmological patient interface 6 is configured to be arranged at an inclination angle α with respect to the optical axis of the eye 91. The ophthalmological patient interface 6 is configured to be arranged laterally next to the optical axis v of the eye 91, at a different position on the almost spherical eyeball. The ophthalmological patient interface 6 extends, due to its unique features, the possible treatment surface 623, which is reachable by the treatment laser beam T, substantially. The ophthalmological laser treatment system is for example configured to treat the sclera of the eye 91 by the treatment laser beam T. The Figures 6 to 8 and 11 further indicate schematically that a coupling liquid is arranged in the through opening 63. The treatment surface 623 is according to these embodiments not deformed by the ophthalmological patient interface 6, which reduces the injury possibility due to the application of the ophthalmological patient interface 6. The Figures 9, 10 and 12 shows the ophthalmological patient interface 6 with an applanating contact body, which is configured to conform / deform the surface of the eye 91.

The Figures 6 to 12 show different embodiments of the ophthalmological patient interface 6 according to the present disclosure. All of these Figures show the treatment laser beam T, the application head 5 and the ophthalmological patient interface 6 applied on the eye 91 of a person 9. The Figures further show the focusing optics 51 associated with the application head 5 and the through opening 63 extending through the coupling portion 61 and the eye fixation portion 62. The focusing optics 51 are for example arranged in the coupling liquid or above the coupling liquid.

The Figures 6 to 12 further show that the eye fixation portion 62 of the ophthalmological laser treatment system 100 comprises a cornea contacting element 622a, configured to contact at least partially the cornea of the eye 91, and a sclera contacting element 622b, configured to contact at least partially the sclera of the eye 91, wherein the cornea contacting element 622a and the sclera contacting element 622b define a rotationally asymmetric contact surface 622 configured to contact the eye 91. The contact surface 622 of the eye fixation potion 62 is rotationally asymmetric such that the eye fixation portion 62 is advantageously dockable / arrangeable to the surface of the eye 91 laterally next to the optical axis v of the eye 91. The Figures show that the human eye 91 has not a perfect spherical shape, the cornea of the eye 91 forms a spherical bulge or a spherical protrusion on the eyeball. The optical axis v of the eye 91 is arranged in line with the center of the bulge and the center of the eyeball. A conventional rotational symmetric patient interface, as shown in the Figures 4 and 5 can therefore only align perfectly on the surface of the eyeball if arranged coaxially with the optical axis v of the eye 91. The rotationally asymmetry of the ophthalmological patient interface 6 according to the present disclosure enables to position the ophthalmological patient interface 6 perfectly aligned on the surface of the eye 91 at any other position, contacting the cornea, via the cornea contacting element 622a and the sclera via the sclera contacting element 622b of the eye 91. The ophthalmological laser treatment system 100 according to the present disclosure enables therefore for example to treat the sclera and the cornea of the eye 91.

**Figure 6** shows a first embodiment of the ophthalmological patient interface 6 used in the ophthalmological laser treatment system 100 according to the present disclosure. The rotational asymmetric contact surface 622 is formed by a sealing 624, which forms part of the sclera contacting element 622b, and by a suction opening 621 of the cornea contacting element 622a. In other words, the sealing 624, for example a balloon sealing, and the cornea contacting element 622a comprising the suction opening 621 form or determine the rotational asymmetric contact surface 622 of the patient interface 6. The sealing 624 is configured to seal between the sclera of the eye 91 and the eye fixation portion 62. The suction opening 621, in particular a housing of the suction opening 621, is configured to be arranged at least partially on the cornea of the eye 91 and to position the ophthalmological patient interface 6 rigidly on the eye 61. The suction opening 621 has for example a partial ring shape. The suction opening 621 is configured to be connected to a negative pressure / vacuum. According to this embodiment, the treatment laser beam T can advantageously treat the sclera of the eye 91. The embodiment of the ophthalmological patient interface 6 as shown in Figure 6 is for example used for a pterygium surgery. The ophthalmological patient interface 6 is for example placed rigidly above the pterygium and the treatment laser beam removes afterwards the pterygium. Further, this embodiment of the ophthalmological patient interface 6 could be used to treat a melanoma of the eye, arranged on the sclera and / or the cornea of the eye in a similar manner as the pterygium treatment. Further, with the ophthalmological patient interface 6 it could be possible to perform a glaucoma surgery from the outside of the eye. The through opening 63 is for example rigidly placed above the schlemm canal (Sinus venosus sclerae). The treatment laser beam T is guided through the sclera and is focused on the schlemm canal to treat the schlemm canal as desired. In another embodiment, a small flap is introduced to the sclera to reach the schlemm canal.

The **Figures 7** and **8** shows a second and third embodiment of the ophthalmological patient interface 6 used in the ophthalmological laser treatment system 100 according to the present disclosure. Figures 7 and 8 differs from Figure 6 in the shape of the eye fixation portion 62 of the ophthalmological patient interface 6, which enables to form a larger inclination angle α between the axis p and the optical axis v of the eye 91, when the ophthalmological patient interface 6 is applied on the eye 91. The rotationally asymmetric contact surface 622 of these embodiments is also formed by a sealing 624, forming the sclera contacting element 622b, and by the cornea contacting element 622a comprising one suction opening 621. The cornea contacting element 622a comprises an extension portion 627, which extends from the coupling portion 61 beyond the maximal extension of the sclera contacting element 622b. The extension portion 627 follows a radius such that the tip of the extension portion 627, which comprises the suction opening 621, is advantageously applicable on the cornea of the eye 91. The embodiments shown in the Figures 7 and 8 differ in the length of the extension portions 627. The length of the extension portion 627 determines the inclination angle α between the axis p of the ophthalmological patient interface 6 and the axis v of the eye 91, when applied on the eye 91. The length of the extension portion 627 therefore also determines the angle of incidence of the treatment laser beam T. In Figure 7, the treatment surface 623, which is advantageous reachable by the treatment laser beam T, using this specific ophthalmological patient interface 6, is the interior side of the ocular limbus of the eye 91. The embodiment of the ophthalmological patient interface 6 as shown in Figure 7 is for example used in a stem cell harvesting surgery. The interior side of the ocular limbus comprises stem cells which can be harvested by the treatment laser beam T using the ophthalmological patient interface 6 as shown in Figure 7.

In Figure 8, the treatment surface 623, which is advantageous reachable by the treatment laser beam T, using this specific ophthalmological patient interface 6, is the anterior chamber angle of the eye 91. The ophthalmological patient interface 6, having different lengths of the extension portion 627 is, for example, selected in dependence of the desired treatment. The embodiment of the ophthalmological patient interface 6 as shown in Figure 8 is for example used in glaucoma surgery to drill holes in the schlemm canal. In this embodiment, the treatment laser beam is guided through the cornea of the eye and focused in the limbus to drill the desired holed in the schlemm canal.

The Figures 9 and 10 show a fourth and fifth embodiment of the ophthalmological patient interface 6 used in the ophthalmological laser treatment system 100 according to the present disclosure. These embodiments comprise a contact body 626, arranged in in the through opening 63. The contact body 626 or contact glass is at least partially transparent for the treatment laser beam T to enable the penetration of the target volume of tissue of the eye 91. The flat contact body 626 comprises a flat contact surface 628, which forms part of the rotationally asymmetric contact surface 622 and which is configured to conform the sclera of the eye 91. These embodiments do not comprise a coupling liquid arranged in the through opening 63 and also do not comprise a corresponding sealing. The fourth embodiment of Figure 9 comprises a suction opening 621, preferably partially ring shaped and preferably partially surrounding the contact body 626, which is arranged in the cornea contacting element 622a. The sclera contacting element 622b is according to this embodiment at least partially formed by the contact body 626 and the cornea contacting element 622a is formed by the part which houses the suction opening 621 and also partially by the contact body 626. The contact body 626 is, according to this embodiment, configured to contact with its flat contact surface 628 the cornea and the sclera of the eye 91.

The fifth embodiment of the ophthalmological patient interface 6 used in the ophthalmological laser treatment system 100 according to the present disclosure shown in Figure 10 comprises at least one protrusion 625, which forms part of the sclera contacting element 622b and which is configured to penetrate tissue of the sclera of the eye 91, when the ophthalmological patient interface 6 is applied on the eye 91. The protrusions 625 are spike shaped and are arranged laterally next to the contact body 626. The protrusions 625 have for example the shape of a pyramid. The protrusions 625 advantageously help to position the ophthalmological patient interface 6 rigidly on the eye 91. Sclera tissue is relatively soft and is not easily damaged by the protrusions 625, compared to cornea tissue. Nevertheless, in a further embodiment, at least one protrusion 625 could be also arranged in the cornea contacting element 622a configured to penetrate the cornea of the eye 91. The protrusions 625 of the cornea contacting element 622a are for example smaller with respect to the protrusions 625 of the sclera contacting element 622b. The protrusions 625 are for example distributed unevenly around the contact surface 622, thereby forming it's rotationally asymmetry. In another embodiment, the protrusions 625 are evenly distributed around the rotationally asymmetric contact surface 622.

In a further embodiment, not shown in the Figures, the eye fixation portion 62 may comprise protrusions 625 and at least one, preferably a plurality of suction openings 621, arranged in the sclera contacting element 622b and / or in the cornea contacting element 622a.

In a further embodiment, not shown in the Figures, the contact body 626 comprises instead of the flat contact surface 628 a spherical contact surface, which corresponds to the outer non-deformed surface of the eye 91, onto which the contact body 626 is configured to be applied. The contact body 626 or the ophthalmological patient interface 6 comprising the contact body 626 is, for example, selected in dependence of the outer surface of the specific eye 91 and / or on the position onto which the ophthalmological patient interface 6 should be applied on the eye 91. This improves the alignment of the ophthalmological patient interface 6 on the eye 91.

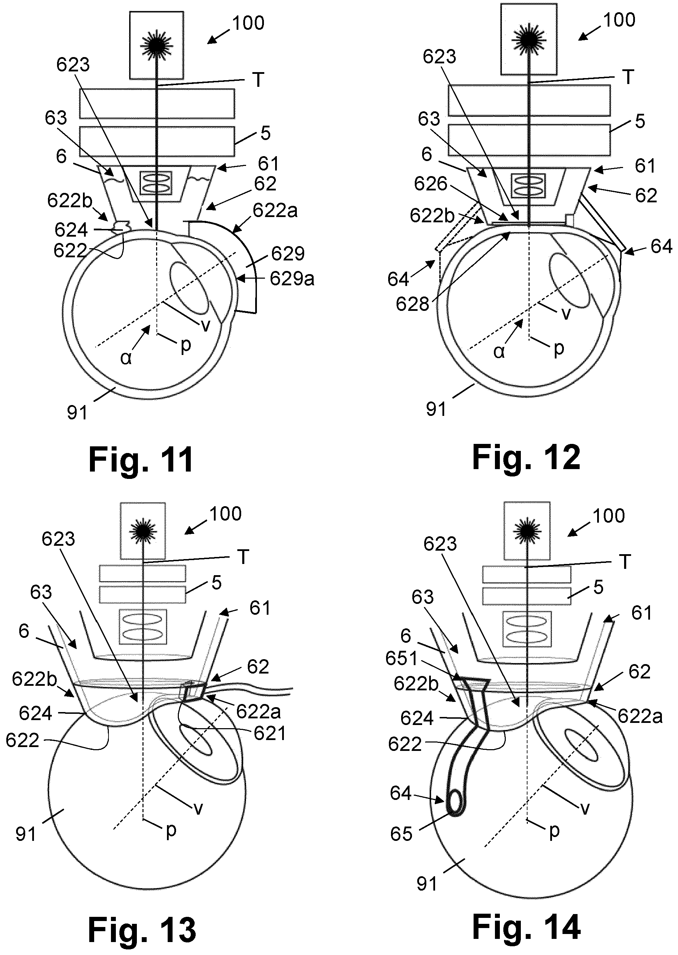

**Figure 11** shows schematically a sixth embodiment of the ophthalmological patient interface 6 used in the ophthalmological laser treatment system 100 according to the present disclosure according to the present disclosure. The rotationally asymmetric contact surface 622 is formed by a sealing 624 forming part of the sclera contacting portion 622b and by a form fitted contact body 629 comprising a form fitted contact surface 629a forming part of the cornea contacting portion 622a. As best visible in Figure 11, the form fitted contact body 629 extends laterally of the ophthalmological patient interface 6 next to the through opening 62 along a shape which corresponds to a negative of a cornea of the eye 91. In other words, the form fitted contact surface 629a, which is configured to contact the cornea of the eye 91, has at least partially the surface shape of the cornea of the eye 91. When applied on the eye, the form fitted contact surface 629a docks advantageously on the cornea of the eye 91, such that the ophthalmological patient interface 6 is advantageously rigidly attached to the eye 91. The surface tension of the tear film provides an advantageous docking of the form-fitted contact body 629 on the eye 91, which advantageously improves the docking of the entire ophthalmological patient interface 6 on the eye 91. In a further embodiment, the form-fitted contact surface 629a corresponds to an individual asphericity of the cornea of the eye 91.

**Figure 12** shows schematically a seventh embodiment of the ophthalmological patient interface 6 used in the ophthalmological laser treatment system 100 according to the present disclosure according to the present disclosure. The rotational asymmetric contact surface 622 is determined by a suction cup 64, which is arranged next to the treatment surface 623 and which is configured to contact the surface of the eye 91 to position the ophthalmological patient interface 6 rigidly on the eye 91. The suction cup 64 is for example a round rubber part. The suction cup 64 is preferably arranged coaxially with the optical axis v of the eye 91 on the cornea. The suction cup 64 is for example arranged on one tip of a bracket, which extends from the coupling portion 61 or the eye fixation portion 62, the other tip or end of the bracket is connected to the coupling portion 61 or the eye fixation portion 62. The bracket, in particular the bracket length and bracket angle determines the position of the suction cup 64 with respect of the rest of the contact surface 622. The ophthalmological patient interface 6 may comprise a plurality of suction cups 64 arranged around the ophthalmological patient interface 6, having for example different brackets, for an optimized positioning of the ophthalmological patient interface 6 on the eye 91.

**Figure 13** shows schematically an eight embodiment of the ophthalmological patient interface 6 used in the ophthalmological laser treatment system 100 according to the present disclosure according to the present disclosure. Figure 13 shows in a perspective view the ophthalmological patient interface 6 applied on the eyeball. Figure 13 advantageously shows the rotational asymmetry of the contact surface 622 of the eye fixation portion 62. The contact surface 622 as shown in this Figure is not only rotational asymmetric in a plane area but also varies in its vertical extension such that the contact surface 622 advantageously fits / advantageously touches the surface of the eye 91. The contact surface 622 according to this embodiment is formed by the eye fixation portion 62, which extends from the coupling portion 61. The extension length varies around the eye fixation portion 62, such that the contact surface 622 is applicable on the sclera and on the cornea, which extends spherical from the spherical sclera. Figure 13 further shows that the eye fixation portion 62, in particular the cornea contacting element 622a, comprises a suction opening 621, which has a partial ring shape and which is connectable via a pressure line to a negative pressure. The suction opening 621 is according to this embodiment configured to be arranged on the cornea of the eye 91. In a further embodiment, the suction opening 621 might extends towards the sclera. In a further embodiment, the eye fixation portion 62, in particular the cornea contacting element 622a and or the sclera contacting element 622b, might comprise one or a plurality of suction openings 621.

**Figure 14** shows schematically a ninth embodiment of the ophthalmological patient interface 6 used in the ophthalmological laser treatment system 100 according to the present disclosure according to the present disclosure. The embodiment as shown in Figure 14 differs from the embodiment as shown in Figure 13 in that the eye fixation portion 62 does not comprise a suction opening. The embodiment as shown in Figure 14 comprises a spoon shaped gripper arm 65. The spoon shaped gripper arm 65 extends from the coupling portion 61 and the eye fixation portion 62 vertically beyond the rest of the contact surface 622. The spoon shaped gripper arm 65 is configured to contact at least partially the eye 91 and forms therefore part of the rotationally asymmetric contact surface 622. The spoon shaped gripper arm 65 extends or follows along an arc, which has an eye-ball radius. In other words, the part of the spoon shaped gripper arm 65 extending beyond the rest of the contact surface 622 follows the form of the eyeball. The spoon shaped gripper arm 65 provides an advantages docking solution for docking of the ophthalmological patient interface 6 on the eye 91. The spoon shaped gripper arm 65 comprises at its tip according to this embodiment an optional suction cup 64. The embodiments of the ophthalmological patient interface 6 show that the eye fixation portion 62 and the coupling portion 61 are two separate parts, which are attached / connected with each other to form the ophthalmological patient interface 6. In the Figures 13 and 14, this separation is indicated by a line between the two parts. Figure 14 further shows that the spoon shaped gripper arm 65 extends along the eye fixation portion 62 and the coupling portion 62. The ophthalmological patient interface 6 as shown in Figure 14 further comprises a safety coupling 651. The safety coupling 651 according to this embodiment is implemented between the coupling portion 61 and the eye fixation portion 62. Both parts are arranged detachable to each other, such that the safety coupling 651 opens, when a force, for example an unexpected hit by a third person, engages on the ophthalmological patient interface 6. The safety coupling 651 is configured such that it opens, when the force, which engages on the ophthalmological patient interface 6 reaches or surpasses a predefined threshold. The safety coupling 651 may comprise magnets and / or a vacuum / negative pressure and / or a spring element.

In a further embodiment, not shown in the Figures, the ophthalmological patient interface 6 comprises a plurality of spoon shaped gripper arms 65, for example, two arranged on opposing sides of the ophthalmological patient interface 6. Having a plurality of the spoon shaped gripper arms 65 advantageously helps to improve the rigid positioning of the ophthalmological patient interface 6 on the eye 91.

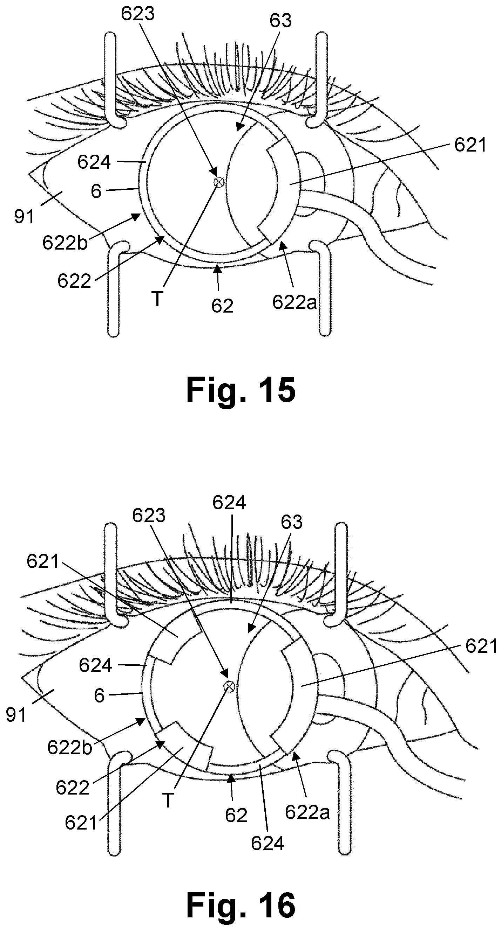

The **Figures 15, 16** and **17** shows schematically a tenth, eleventh and twelfth embodiment of the ophthalmological patient interface 6 used in ophthalmological laser treatment system 100 according to the present disclosure. All of these Figures show a top view of the ophthalmological patient interface 6 arranged on the eye 91. The Figures do not show the entire ophthalmological patient interface 6, but only show the contact surface 622 of the ophthalmological patient interface 6. The Figures 15 to 17 advantageously show the rotational asymmetry of the contact surface 622. Figure 15 and Figure 16 shows that the contact surface 622 has a circular outer contour. Figure 15 further show that the contact surface 622 comprises a suction opening 621 arranged in the cornea contacting element 622a, which determines the rotational asymmetry of the contact surface. The suction opening 621 as shown in Figurer 15 is arranged only in the cornea contacting element 622a. The sclera contacting element 622b of this embodiment does not comprise any suction opening. The contact surface 622 of the embodiment as shown in Figure 15 further comprises a sealing 624, which is arranged mainly on the sclera contacting element 622b but also extends to the cornea contacting element 622a. The contact surface 622 is determined according to this embodiment by the sealing 624 and the suction opening 621.

**Figure 16** differs from Figure 15 in that it has a plurality of suction openings 621. One is arranged in the cornea contacting element 622a and two are arranged in the sclera contacting element 622b. According to this embodiment, a sealing 624 is arranged between the suction openings 621. The negative pressure applied on the suction opening 621 of the cornea contacting element 622a is in an embodiment higher compared to the negative pressure applied on the suction openings 621 of the sclera contacting elements 622b. This is for example achieved via different negative pressure lines providing different negative pressures on the suction openings 621.

**Figure 17** differs from Figure 16 in that the contact surface 622 of the ophthalmological patient interface 6 as shown in Figure 17 does not have a circular outer contour. The outer contour of the contact surface 622 has an egg shape or has an oval shape.

The fourth to twelfth embodiments of the ophthalmological patient interface 6 as shown in the Figures 9 to 17 are for example used by the ophthalmological laser treatment system 100 in a pterygium surgery, in a melanoma surgery or in a glaucoma surgery as explained in detail with reference to Figure 6 above. All of these embodiments enable similarly advantageously to place the through opening 63 above at least partially the sclera of the eye 91, such that the above-mentioned eye-treatment surgeries can be performed advantageously by the treatment laser beam T.

In a further embodiment, not shown in the Figures, the ophthalmological laser treatment system 100 further comprises a liquid dispenser, which is configured to dispense continuously or on demand coupling liquid into the through opening 63. The ophthalmological patient interface 6 may comprise an opening, for example, in the eye fixation portion 62 and / or in the coupling portion 62, which is configured to supply / dispense the coupling liquid from the liquid dispenser into the through opening 63.

**Figure 18** shows schematically thirteenth embodiment of the ophthalmological laser treatment system 100. According to this embodiment, the ophthalmological laser treatment system 100 comprises the optical mirror element 10 and an optical imaging device 7. The optical mirror element 10 is arranged in the through opening 63 of the ophthalmological patient interface 6. The optical mirror element 10 is for example attached at the ophthalmological patient interface 6 or at the application head 5. The optical mirror element 10 may form part of the focusing optics 51. The optical mirror element 10 is arranged in the beam path of the treatment laser beam T, comprises a mirror, which is configured to deflect the treatment laser beam T incidenting from the application head 5. Figure 18 shows that the treatment laser beam T is deflected by the mirror element 10 towards the interior side of the ocular limbus of the eye 91. The embodiment of the ophthalmological patient interface 6 as shown in Figure 18 is for example used in a stem cell harvesting surgery. The interior side of the ocular limbus comprises stem cells, which can be harvested by the treatment laser beam T using the ophthalmological patient interface 6 as shown in Figure 18.

The optical mirror element 10 comprises a mirror, which is configured to deflect the treatment laser beam T. The mirror is in one embodiment rigidly connected to the application head 5. In a further embodiment, the mirror element 10 comprises at least one adjustable mirror, which is configured to adjust the specific angle based on a position of the tissue of the eye 91 to be treated and the position of the ophthalmological patient interface 6 on the sclera and cornea of the eye 91 to optimize the angle of incidence of the treatment laser beam T on the eye 91. With the adjustable mirror, it is possible to optimize the angle of incidence of the treatment laser beam T on the eye 91. The mirror is for example adjusted automatically or manually.

The optical mirror element 10 is for example implemented in the application head 5, in particular in the focusing optics 51 of the application head 5.

Figure 18 further shows schematically the optical imaging device 7, which is configured to generate optical image data of the eye 91. The optical image data is used for positioning of the ophthalmological patient interface 6 on the sclera and cornea of the eye 91. Further, the optical image data could additionally or alternatively be used for adapting of the position of the ophthalmological patient interface 6 on the sclera and cornea of the eye 91, for example in case the position is not perfectly aligned from the beginning.

The optical imaging device 7 is for example implemented in the application head 5, such that the beam path of the optical imaging device 7 follows the beam path of the treatment laser beam T, such that the optical imaging device 7 is configured to provide optical image data of the treatment surface 63. The optical imaging device 7 is for example also configured to "follow" the deflection by the optical mirror element. The optical imaging device 7 may comprise a camera.

The optical imaging device 7 as shown in Figure 18 may comprises an optical depth module 71, which is configured to provide depth information of the eye 91. The depth information are additionally or alternatively used for positioning of the ophthalmological patient interface 6 on the sclera and cornea of the eye 91 and / or for adapting of the position of the ophthalmological patient interface 6 on the sclera and cornea of the eye 91. The depth module 71 may comprise an imaging device based on the Scheimpflug principle, an optical coherence tomography device, a confocal microscopy device, a device for structured lighting and / or a device for stereoscopic vision.

Figure 18 further shows schematically the control unit 3, which is configured to control the treatment laser beam T. Figure 18 shows that the optical data is transmitted from the optical imaging device 7 to the control unit 3.

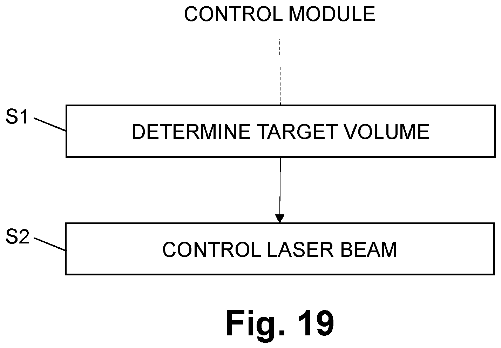

Figure 19 shows a flow chart of some steps performed by the control module 10 to control the treatment laser beam T of the ophthalmological laser treatment system 100.

In step S1, the target volume of tissue to be treated is determined by a processing unit of the control module 3 using the captured image data and / or the depth information, of the optical imaging device 7. The data is transmitted from the optical imaging device 7 to the control module 3 for processing. The control module 3 determines for example the extension of the volume to be treated, for example the 3D extension of a pterygium, using the optical data and the depth information.

In step S2, the treatment laser beam T is controlled by the processing unit of the control module 3 for penetration of the target volume, using the determined target volume of tissue to be treated of step S1. The processing unit determines for example control instructions for different parts (scanner 22, focusing optics 51, mirror element 10) of the ophthalmological laser treatment system 100 in order to control the treatment laser beam T. The control module 3 transmits these control instructions to the respective parts to control the treatment laser beam T as desired.

For example, based on the 3D extension of the pterygium, a specific cutting pattern is determined by the control module 3. In a further step, the treatment laser beam T is controlled such that it follows the determined specific cutting pattern. In a further step, a control loop is implemented using continuously updated data from the optical imaging device 7 for an iterative control of the treatment laser beam T.

### LIST OF REFERENCE SIGNS:

- 100: ophthalmological laser treatment system
- 1: ophthalmological laser treatment device
- 2: base station
- 21: treatment laser source
- 22: scanner
- 23: optical module
- 3: control module
- 4: arm
- 41: first arm joint
- 42: second arm joint
- 43: third arm joint
- 5: application head
- 6: patient interface
- 61: coupling portion
- 62: eye fixation portion
- 621: suction opening
- 622: rotationally asymmetric contact surface
- 622a: cornea contacting element
- 622b: sclera contacting element
- 623: treatment surface
- 624: sealing
- 625: protrusion
- 626: contact body
- 627: extension portion
- 628: flat contact surface
- 629: form-fitted contact body
- 629a: form-fitted contact surface
- 63: through opening
- 64: suction cup
- 65: spoon-shaped gripper arm
- 651: safety coupling
- 7: optical imaging device
- 71: depth module
- 8: monitor
- 9: person
- 91: eye of the person
- 10: optical mirror element

- T: treatment laser beam
- v: optical axis of an eye
- p: main central optical axis of the application head
- r: central axis of the coupling portion
- c: central axis of the eye fixation portion
- α: inclination angle

## Claims

1. An ophthalmological laser treatment system (100) for treatment of tissue of an eye (91) of a person (9), the ophthalmological laser treatment system (100) comprising:
a. an ophthalmological laser treatment device (1) comprising a base station (2) having a treatment laser source (21) configured to generate a treatment laser beam (T), an arm (4) and an application head (5), wherein the arm (4) is configured to guide the treatment laser beam (T) from the base station (2) to the application head (5),
b. an ophthalmological patient interface (6), the ophthalmological patient interface (6) comprising, a coupling portion (61), configured to be arranged on the application head (5), and an eye fixation portion (62), configured to be arranged on the eye (91), wherein the eye fixation portion (62) comprises a cornea contacting element (622a), configured to contact at least partially the cornea of the eye (91), and a sclera contacting element (622b), configured to contact at least partially the sclera of the eye (91), for firmly positioning the ophthalmological patient interface (6) on the eye (91), wherein the cornea contacting element (622a) and the sclera contacting element (622b) define a rotationally asymmetric contact surface (622) configured to contact the eye (91) and configured to apply the ophthalmological patient interface (6) non-coaxially with respect to the optical axis (v) of the eye (91), and
wherein the ophthalmological patient interface (6) comprises a through opening (63), extending through the coupling portion (61) and the eye fixation portion (62), wherein the through opening (63) is configured to enable the treatment laser beam (T) from the application head (5) to pass through the ophthalmological patient interface (6) to penetrate a target volume of tissue of the eye (91).

2. The ophthalmological laser treatment system (100) according to claim 1, further comprising an optical mirror element (10) arranged between the application head (5) and the ophthalmological patient interface (6) or within the ophthalmological patient interface (6), wherein the optical mirror element (10) is configured to deflect the treatment laser beam (T) incidenting from the application head (5) at a specific angle, wherein the angle of incidence of the treatment laser beam (T) on the eye (91) with respect to an optical axis (v) of the eye (91) is determined by the inclined position of the ophthalmological patient interface (6) with respect to the optical axis (v) on the eye (91) and the specific angle of the optical mirror element (10).

3. The ophthalmological laser treatment system (100) according to claim 2, wherein the optical mirror element (10) comprises at least one adjustable mirror, which is configured to adjust the specific angle based on a position of the tissue of the eye (91) to be treated and the position of the ophthalmological patient interface (6) on the sclera and cornea of the eye (91) to adjust the angle of incidence of the treatment laser beam (T) on the eye (91).

4. The ophthalmological laser treatment system (100) according to one of the preceding claims, further comprising an optical imaging device (7) configured to generate optical image data of the eye (91) of the person (9), which are configured to be used for positioning of the ophthalmological patient interface (6) on the sclera and cornea of the eye (91) and / or for adapting of the position of the ophthalmological patient interface (6) on the sclera and cornea of the eye (91).

5. The ophthalmological laser treatment system (100) according to claim 4, wherein the optical imaging device (7) further comprises an optical depth module (71), which is configured to provide depth information of the eye (91), which are configured to be used for positioning of the ophthalmological patient interface (6) on the sclera and cornea of the eye (91) and / or for adapting of the position of the ophthalmological patient interface (6) on the sclera and cornea of the eye (91).

6. The ophthalmological laser treatment system (100) according to claim 5, wherein the optical depth module (71), which is configured to provide depth information of the eye (91), is an imaging device based on the Scheimpflug principle, an optical coherence tomography device, a confocal microscopy device, a device for structured lighting and / or a device for stereoscopic vision.

7. The ophthalmological laser treatment system (100) according to one of the claims 4 to 6, wherein the ophthalmological laser treatment system (100) further comprises a control module (3), wherein the control module (3) is configured to:
a. determine (S1), by a processing unit of the control module (3), the target volume of tissue to be treated using at least one of: the captured image data or the depth information, of the optical imaging device (7), and
b. control (S2), by the processing unit, the treatment laser beam (T) for penetration of the target volume, using the determined target volume of tissue to be treated.

8. The ophthalmological laser treatment system (100) according to one of the preceding claims, wherein the cornea contacting element (622a) comprises a rotationally asymmetric suction opening (621) configured to be positioned at least partially, preferably entirely, on the cornea of the eye (91) and configured to be fluidically connected to a negative pressure, and / or wherein the sclera contacting element (622b) comprises a rotationally asymmetric suction opening (621) configured to be positioned at least partially, preferably entirely, on the sclera of the eye (91) and configured to be fluidically connected to a negative pressure for positioning the ophthalmological patient interface (6) on the eye (91).

9. The ophthalmological laser treatment system (100) according to one of the preceding claims, wherein the through opening (63) is configured to be at least partially filled with a coupling liquid, wherein at least one of: the cornea contacting element (622a) or the sclera contacting element (622b) comprises a sealing (624), and wherein the sealing (624) is configured to seal at least partially between the surface of the eye (91) and the ophthalmological patient interface (6) when the ophthalmological patient interface (6) is applied on the eye (91) and the coupling liquid is arranged in the through opening (63).

10. The ophthalmological laser treatment system (100) according to one of the preceding claims, wherein the eye fixation portion (62) comprises a flat contact body (626) arranged in in the through opening (63), wherein the contact body (626) is at least partially transparent for the treatment laser beam (T) to enable the penetration of the target volume of tissue of the eye (91), wherein the flat contact body (626) comprises a flat contact surface, which forms part of the rotationally asymmetric contact surface (622) and which is configured to conform the sclera of the eye (91).

11. The ophthalmological laser treatment system (100) according to one of the preceding claims, wherein the cornea contacting element (622a) comprises a form-fitted contact body (629) comprising a form-fitted contact surface (629a) forming part of the rotationally asymmetric contact surface (622), wherein the form-fitted contact surface (629a) is form-fitted to a shape of the outer surface of the cornea of the eye (91), wherein the form-fitted contact surface (629a) is configured to be at least partially in form-fitting contact with the cornea of the eye (91) for firmly positioning the ophthalmological patient interface (6) on the eye (91).

12. The ophthalmological laser treatment system (100) according to one of the preceding claims, wherein at least one of: the cornea contacting element (622a) or the sclera contacting element (622b) comprises protrusions (625), which are configured to penetrate tissue of the sclera or the cornea respectively.

13. The ophthalmological laser treatment system (100) according to one of the preceding claims, wherein at least one of: the cornea contacting element (622a) or the sclera contacting element (622b) comprise at least one suction cup (64) having a flexible contact body configured to contact the eye (91), thereby forming part of the rotationally asymmetric contact surface (622), for positioning the ophthalmological patient interface (6) with respect to the eye (91).

14. The ophthalmological laser treatment system (100) according to claim 13, wherein the ophthalmological patient interface (6) further comprises a bracket arranged between the suction cup (64) and the eye fixation portion (62), wherein the bracket extends from the eye fixation portion (62) at a specific angle and at a specific length such that the suction cup (64) is configured to contact the cornea of the eye (91) coaxially with respect to the optical axis (v) of the eye (91).

15. The ophthalmological laser treatment system (100) according to one of the preceding claims, wherein the sclera contacting element (622b) comprises at least one, preferably a plurality of, spoon-shaped gripper arm(s) (65) extending from the coupling portion (61) and / or the eye fixation portion (62), wherein the spoon-shaped gripper arm(s) (65) extend arc-shaped having an eye-ball radius from the eye fixation portion (62), thereby forming part of the rotationally asymmetric contact surface (622), for positioning the ophthalmological patient interface (6) with respect to the eye (91).

## Patentansprüche

1. Ein ophthalmologisches Laserbehandlungssystem (100) zur Behandlung von Gewebe eines Auges (91) einer Person (9), wobei das ophthalmologische Laserbehandlungssystem (100) umfasst:
a. eine ophthalmologische Laserbehandlungsvorrichtung (1), umfassend eine Basisstation (2) mit einer Behandlungslaserquelle (21), die zur Erzeugung eines Behandlungslaserstrahls (T) eingerichtet ist, einen Arm (4) und einen Applikationskopf (5), wobei der Arm (4) eingerichtet ist, den Behandlungslaserstrahl (T) von der Basisstation (2) zu dem Applikationskopf (5) zu leiten,
b. eine ophthalmologische Patientenschnittstelle (6), die ophthalmologische Patientenschnittstelle (6) umfassend einen Kopplungsabschnitt (61), der eingerichtet ist, dass er am Applikationskopf (5) angeordnet werden kann, und einen Augenfixierungsabschnitt (62), der eingerichtet ist, dass er am Auge (91) angeordnet werden kann, wobei der Augenfixierungsabschnitt (62) ein Hornhautkontaktelement (622a) umfasst, das eingerichtet ist, dass es zumindest teilweise die Hornhaut des Auges (91) berührt, sowie ein Sklerakontaktelement (622b), das eingerichtet ist, dass es zumindest teilweise die Sklera des Auges (91) berührt, um die ophthalmologische Patientenschnittstelle (6) fest auf dem Auge (91) zu positionieren, wobei das Hornhautkontaktelement (622a) und das Sklerakontaktelement (622b) eine rotationsasymmetrische Kontaktfläche (622) definieren, eingerichtet das Auge (91) zu kontaktieren und eingerichtet, dass die ophthalmologische Patientenschnittstelle (6) nicht koaxial zur optischen Achse (v) des Auges (91) angelegt ist, und
wobei die ophthalmologische Patientenschnittstelle (6) eine Durchgangsöffnung (63) umfasst, die sich durch den Kupplungsabschnitt (61) und den Augenfixierungsabschnitt (62) erstreckt, wobei die Durchgangsöffnung (63) eingerichtet ist dem Behandlungslaserstrahl (T) von dem Applikationskopf (5) zu ermöglichen, durch die ophthalmologische Patientenschnittstelle (6) hindurchzutreten, um in ein Zielgewebevolumen des Auges (91) einzudringen.

2. Das ophthalmologische Laserbehandlungssystem (100) nach Anspruch 1, das ferner ein optisches Spiegelelement (10) umfasst, das zwischen dem Applikationskopf (5) und der ophthalmologischen Patientenschnittstelle (6) oder innerhalb der ophthalmologischen Patientenschnittstelle (6) angeordnet ist, wobei das optische Spiegelelement (10) so eingerichtet ist, dass es den von dem Applikationskopf (5) einfallenden Behandlungsstrahl (T) in einem bestimmten Winkel ablenkt, wobei der Einfallswinkel des Behandlungslaserstrahls (T) auf das Auge (91) in Bezug auf eine optische Achse (v) des Auges (91) durch die geneigte Position der ophthalmologischen Patientenschnittstelle (6) in Bezug auf die optische Achse (v) des Auges (91) und dem bestimmten Winkel des optischen Spiegelelements (10) bestimmt wird.

3. Das ophthalmologische Laserbehandlungssystem (100) nach Anspruch 2, wobei das optische Spiegelelement (10) mindestens einen verstellbaren Spiegel umfasst, der eingerichtet ist, dass er den spezifischen Winkel auf der Grundlage einer Position des zu behandelnden Augengewebes (91) und der Position der ophthalmologischen Patientenschnittstelle (6) auf der Sklera und der Hornhaut des Auges (91) einstellt, um den Einfallswinkel des Behandlungslaserstrahls (T) auf das Auge (91) anzupassen.

4. Das ophthalmologische Laserbehandlungssystem (100) gemäß einem der vorstehenden Ansprüche, das ferner eine optische Bildgebungsvorrichtung (7) umfasst, die eingerichtet ist, dass sie optische Bilddaten des Auges (91) der Person (9) erzeugt, die zur Positionierung der ophthalmologischen Patientenschnittstelle (6) auf der Sklera und der Hornhaut des Auges (91) und/oder zur Anpassung der Position der ophthalmologischen Patientenschnittstelle (6) auf der Sklera und der Hornhaut des Auges (91) verwendet werden können.

5. Das ophthalmologische Laserbehandlungssystem (100) gemäß Anspruch 4, wobei die optische Bildgebungsvorrichtung (7) ferner ein optisches Tiefenmodul (71) umfasst, das eingerichtet ist, Tiefeninformationen des Auges (91) bereitzustellen, die zur Positionierung der ophthalmologischen Patientenschnittstelle (6) auf der Sklera und der Hornhaut des Auges (91) und/oder zur Anpassung der Position der ophthalmologischen Patientenschnittstelle (6) auf der Sklera und der Hornhaut des Auges (91) verwendet werden.

6. Das ophthalmologische Laserbehandlungssystem (100) gemäß Anspruch 5, wobei das optische Tiefenmodul (71), das eingerichtet ist Tiefeninformationen des Auges (91) bereitzustellen, eine auf dem Scheimpflug-Prinzip basierende Abbildungsvorrichtung, eine optische Kohärenztomographie-Vorrichtung, eine Konfokalmikroskopie-Vorrichtung, eine Vorrichtung für strukturierte Beleuchtung und/oder eine Vorrichtung für stereoskopisches sehen ist.

7. Das ophthalmologische Laserbehandlungssystem (100) gemäß einem der Ansprüche 4 bis 6, wobei das ophthalmologische Laserbehandlungssystem (100) ferner ein Steuermodul (3) umfasst, wobei das Steuermodul (3) zur:
a. Bestimmung (S1), durch eine Verarbeitungseinheit des Steuermoduls (3), des Zielvolumens des zu behandelnden Gewebes unter Verwendung mindestens eines der folgenden: der erfassten Bilddaten oder der Tiefeninformationen der optischen Bildgebungsvorrichtung (7), und
b. Steuerung (S2), durch die Verarbeitungseinheit, des Behandlungslaserstrahls (T) für das Durchdringen des Zielvolumens unter Verwendung des ermittelten Zielvolumens des zu behandelnden Gewebes,
eingerichtet ist.

8. Das ophthalmologische Laserbehandlungssystem (100) gemäß einem der vorstehenden Ansprüche, wobei das Hornhautkontaktelement (622a) eine rotationsasymmetrische Saugöffnung (621) umfasst, die so eingerichtet ist, dass sie zumindest teilweise, vorzugsweise vollständig, auf der Hornhaut des Auges (91) positionierbar ist, und die eingerichtet ist, dass sie fluidisch mit einem Unterdruck verbindbar ist, und/oder wobei das Sklerakontaktelement (622b) eine rotationsasymmetrische Saugöffnung (621) umfasst, die eingerichtet ist, dass sie zumindest teilweise, vorzugsweise vollständig, auf der Sklera des Auges (91) positionierbar ist, und die eingerichtet ist, dass sie fluidisch mit einem Unterdruck verbindbar ist, um die ophthalmologische Patientenschnittstelle (6) auf dem Auge (91) zu positionieren.

9. Das ophthalmologische Laserbehandlungssystem (100) gemäß einem der vorstehenden Ansprüche, wobei die Durchgangsöffnung (63) eingerichtet ist, dass sie zumindest teilweise mit einer Kupplungsflüssigkeit befüllbar ist, wobei mindestens eines von: dem Hornhautkontaktelement (622a) oder dem Sklerakontaktelement (622b) eine Dichtung (624) aufweist, und wobei die Dichtung (624) eingerichtet ist zumindest teilweise zwischen der Oberfläche des Auges (91) und der ophthalmologischen Patientenschnittstelle (6) abzudichten, wenn die ophthalmologische Patientenschnittstelle (6) auf das Auge (91) aufgebracht ist und die Kupplungsflüssigkeit in der Durchgangsöffnung (63) angeordnet ist.

10. Das ophthalmologische Laserbehandlungssystem (100) gemäß einem der vorstehenden Ansprüche, wobei der Augenfixierungsabschnitt (62) einen flachen Kontaktkörper (626) umfasst, der in der Durchgangsöffnung (63) angeordnet ist, wobei der Kontaktkörper (626) für den Behandlungslaserstrahl (T) zumindest teilweise transparent ist, um das Eindringen in das Zielgewebevolumen des Auges (91) zu ermöglichen, wobei der flache Kontaktkörper (626) eine flache Kontaktfläche umfasst, die Teil der rotationsasymmetrischen Kontaktfläche (622) ist und eingerichtet ist sich an die Sklera des Auges (91) anzuschmiegen.

11. Das ophthalmologische Laserbehandlungssystem (100) gemäß einem der vorstehenden Ansprüche, wobei das Hornhautkontaktelement (622a) einen formangepassten Kontaktkörper (629) umfasst, der eine formangepasste Kontaktfläche (629a) aufweist, die einen Teil der rotationsasymmetrischen Kontaktfläche (622) bildet, wobei die formangepasste Kontaktfläche (629a) an die Form der Außenfläche der Hornhaut des Auges (91) angepasst ist, wobei die formangepasste Kontaktfläche (629a) eingerichtet ist, dass sie zumindest teilweise in formangepasstem Kontakt mit der Hornhaut des Auges (91) steht, um die ophthalmologische Patientenschnittstelle (6) fest auf dem Auge (91) zu positionieren.

12. Das ophthalmologische Laserbehandlungssystem (100) gemäß einem der vorstehenden Ansprüche, wobei mindestens eines von: dem Hornhautkontaktelement (622a) oder dem Sklerakontaktelement (622b) Vorsprünge (625) aufweist, die so eingerichtet sind, dass sie in Gewebe der Sklera bzw. der Hornhaut eindringen.

13. Das ophthalmologische Laserbehandlungssystem (100) gemäß einem der vorstehenden Ansprüche, wobei mindestens eines von: dem Hornhautkontaktelement (622a) oder dem Sklerakontaktelement (622b) mindestens einen Saugnapf (64) mit einem flexiblen Kontaktkörper aufweist, der so eingerichtet ist, dass er das Auge (91) berührt, wodurch es einen Teil der rotationsasymmetrischen Kontaktfläche (622) zur Positionierung der ophthalmologische Patientenschnittstelle (6) in Bezug auf das Auge (91) bildet.

14. Das ophthalmologische Laserbehandlungssystem (100) gemäß Anspruch 13, wobei die ophthalmologische Patientenschnittstelle (6) ferner eine Halterung umfasst, die zwischen dem Saugnapf (64) und dem Augenfixierungsabschnitt (62) angeordnet ist, wobei sich die Halterung von dem Augenfixierungsabschnitt (62) in einem bestimmten Winkel und über eine bestimmte Länge erstreckt, so dass der Saugnapf (64) so eingerichtet ist, dass er die Hornhaut des Auges (91) koaxial zur optischen Achse (v) des Auges (91) berührt.

15. Das ophthalmologische Laserbehandlungssystem (100) gemäß einem der vorstehenden Ansprüche, wobei das Sklerakontaktelement (622b) mindestens einen, vorzugsweise mehrere, löffelförmige Greifarme (65) umfasst, die sich von dem Kupplungsabschnitt (61) und/oder dem Augenfixierungsabschnitt (62) erstrecken, wobei sich die löffelförmigen Greifarme (65) bogenförmig mit einem Augenballradius von dem Augenfixierungsabschnitt (62) erstrecken und dadurch einen Teil der rotationsasymmetrischen Kontaktfläche (622) zur Positionierung der ophthalmologische Patientenschnittstelle (6) in Bezug auf das Auge (91) bildet.

## Revendications

1. Un système de traitement ophtalmologique au laser (100) destiné au traitement des tissus de l'œil (91) d'une personne (9), le système de traitement ophtalmologique au laser (100) comprenant :
a. un dispositif de traitement ophtalmologique au laser (1) comprenant une station de base (2) dotée d'une source laser de traitement (21) configurée pour générer un faisceau laser de traitement (T), un bras (4) et une tête d'application (5), dans lequel le bras (4) est configuré pour guider le faisceau laser de traitement (T) depuis la station de base (2) vers la tête d'application (5),
b. une interface patient ophtalmologique (6), l'interface patient ophtalmologique (6) comprenant une partie de couplage (61), configurée pour être disposée sur la tête d'application (5), et une partie de fixation oculaire (62), configurée pour être disposée sur l'œil (91), dans laquelle la partie de fixation oculaire (62) comprend un élément de contact avec la cornée (622a), configuré pour entrer en contact au moins partiellement avec la cornée de l'œil (91), et un élément de contact avec la sclère (622b), configuré pour entrer en contact au moins partiellement avec la sclère de l'œil (91), afin de positionner fermement l'interface patient ophtalmologique (6) sur l'œil (91), dans lequel l'élément de contact avec la cornée (622a) et l'élément de contact avec la sclère (622b) définissent une surface de contact (622) asymétrique en rotation, configurée pour entrer en contact avec l'œil (91) et configurée pour appliquer l'interface patient ophtalmologique (6) de manière non coaxiale par rapport à l'axe optique (v) de l'œil (91) , et
dans lequel l'interface patient ophtalmologique (6) comprend une ouverture traversante (63), s'étendant à travers la partie de couplage (61) et la partie de fixation oculaire (62), dans lequel l'ouverture traversante (63) est configurée pour permettre au faisceau laser de traitement (T) provenant de la tête d'application (5) de traverser l'interface patient ophtalmologique (6) afin de pénétrer dans un volume cible de tissu de l'œil (91).

2. Le système de traitement ophtalmologique au laser (100) selon la revendication 1, comprenant en outre un élément de miroir optique (10) disposé entre la tête d'application (5) et l'interface patient ophtalmologique (6) ou à l'intérieur de l'interface patient ophtalmologique (6), dans lequel l'élément de miroir optique (10) est configuré pour dévier le faisceau laser de traitement (T) incident provenant de la tête d'application (5) selon un angle spécifique, l'angle d'incidence du faisceau laser de traitement (T) sur l'œil (91) par rapport à un axe optique (v) de l'œil (91) étant déterminé par la position inclinée de l'interface patient ophtalmologique (6) par rapport à l'axe optique (v) de l'œil (91) et par l'angle spécifique de l'élément miroir optique (10).

3. Le système de traitement ophtalmologique au laser (100) selon la revendication 2, dans lequel l'élément de miroir optique (10) comprend au moins un miroir réglable, qui est configuré pour ajuster l'angle spécifique en fonction de la position du tissu oculaire (91) à traiter et de la position de l'interface patient ophtalmologique (6) sur la sclère et la cornée de l'œil (91) afin d'ajuster l'angle d'incidence du faisceau laser de traitement (T) sur l'œil (91).

4. Le système de traitement ophtalmologique au laser (100) selon l'une des revendications précédentes, comprenant en outre un dispositif d'imagerie optique (7) configuré pour générer des données d'image optique de l'œil (91) de la personne (9), qui sont configurées pour être utilisées pour le positionnement de l'interface patient ophtalmologique (6) sur la sclère et la cornée de l'œil (91) et/ou pour l'adaptation de la position de l'interface patient ophtalmologique (6) sur la sclère et la cornée de l'œil (91).

5. Le système de traitement ophtalmologique au laser (100) selon la revendication 4, dans lequel le dispositif d'imagerie optique (7) comprend en outre un module de profondeur optique (71), qui est configuré pour fournir des informations de profondeur de l'œil (91), lesquelles sont configurées pour être utilisées pour le positionnement de l'interface patient ophtalmologique (6) sur la sclère et la cornée de l'œil (91) et/ou pour l'ajustement de la position de l'interface patient ophtalmologique (6) sur la sclère et la cornée de l'œil (91).

6. Le système de traitement ophtalmologique au laser (100) selon la revendication 5, dans lequel le module de profondeur optique (71), qui est configuré pour fournir des informations de profondeur de l'œil (91), est un dispositif d'imagerie basé sur le principe de Scheimpflug, un dispositif de tomographie par cohérence optique, un dispositif de microscopie confocale, un dispositif d'éclairage structuré et/ou un dispositif de vision stéréoscopique.

7. Le système de traitement ophtalmologique au laser (100) selon l'une des revendications 4 à 6, dans lequel le système de traitement ophtalmologique au laser (100) comprend en outre un module de commande (3), le module de commande (3) étant configuré pour :
a. déterminer (S1), par une unité de traitement du module de commande (3), le volume cible de tissu à traiter en utilisant au moins l'un des suivants : les données d'image capturées ou les informations de profondeur, provenant du dispositif d'imagerie optique (7), et
b. commander (S2), par l'unité de traitement, le faisceau laser de traitement (T) pour la pénétration du volume cible, en utilisant le volume cible déterminé de tissu à traiter.

8. Le système de traitement ophtalmologique au laser (100) selon l'une des revendications précédentes, dans lequel l'élément de contact avec la cornée (622a) comprend une ouverture d'aspiration asymétrique en rotation (621), configurée pour être positionnée au moins partiellement, de préférence entièrement, sur la cornée de l'œil (91) et configurée pour être reliée fluidiquement à une pression négative, et/ou dans lequel l'élément de contact avec la sclère (622b) comprend une ouverture d'aspiration asymétrique en rotation (621) configurée pour être positionnée au moins partiellement, de préférence entièrement, sur la sclère de l'œil (91) et configurée pour être reliée fluidiquement à une pression négative afin de positionner l'interface patient ophtalmologique (6) sur l'œil (91).

9. Le système de traitement ophtalmologique au laser (100) selon l'une des revendications précédentes, dans lequel l'ouverture traversante (63) est configurée pour être au moins partiellement remplie d'un liquide de couplage, dans lequel au moins l'un des suivants : l'élément de contact avec la cornée (622a) ou l'élément de contact avec la sclère (622b) comprend un joint d'étanchéité (624), et dans lequel le joint d'étanchéité (624) est configuré pour assurer au moins partiellement l'étanchéité entre la surface de l'œil (91) et l'interface patient ophtalmologique (6) lorsque l'interface patient ophtalmologique (6) est appliquée sur l'œil (91) et que le liquide de couplage est disposé dans l'ouverture traversante (63).

10. Le système de traitement ophtalmologique au laser (100) selon l'une des revendications précédentes, dans lequel la partie de fixation oculaire (62) comprend un corps de contact plat (626) disposé dans l'ouverture traversante (63), dans lequel le corps de contact (626) est au moins partiellement transparent au faisceau laser de traitement (T) afin de permettre la pénétration dans le volume cible de tissu de l'œil (91), dans lequel le corps de contact plat (626) comprend une surface de contact plate, qui fait partie de la surface de contact asymétrique en rotation (622) et qui est configurée pour épouser la sclère de l'œil (91).

11. Le système de traitement ophtalmologique au laser (100) selon l'une des revendications précédentes, dans lequel l'élément de contact avec la cornée (622a) comprend un corps de contact adapté à la forme (629) comprenant une surface de contact adaptée à la forme (629a) faisant partie de la surface de contact asymétrique en rotation (622), dans lequel la surface de contact adaptée à la forme (629a) est adaptée à la forme de la surface externe de la cornée de l'œil (91), dans lequel la surface de contact adaptée (629a) est configurée pour être au moins partiellement en contact adapté avec la cornée de l'œil (91) afin de positionner fermement l'interface patient ophtalmologique (6) sur l'œil (91).

12. Le système de traitement ophtalmologique au laser (100) selon l'une des revendications précédentes, dans lequel au moins l'un des suivants : l'élément de contact avec la cornée (622a) ou l'élément de contact avec la sclère (622b) comprend des saillies (625), qui sont configurées pour pénétrer respectivement dans le tissu de la sclère ou de la cornée.

13. Le système de traitement ophtalmologique au laser (100) selon l'une des revendications précédentes, dans lequel au moins l'un des suivants : l'élément de contact avec la cornée (622a) ou l'élément de contact avec la sclère (622b) comprend au moins une ventouse (64) ayant un corps de contact flexible configuré pour entrer en contact avec l'œil (91), formant ainsi une partie de la surface de contact asymétrique en rotation (622), pour positionner l'interface patient ophtalmologique (6) par rapport à l'œil (91).

14. Le système de traitement ophtalmologique au laser (100) selon la revendication 13, dans lequel l'interface patient ophtalmologique (6) comprend en outre un support disposé entre la ventouse (64) et la partie de fixation oculaire (62), dans lequel le support s'étend à partir de la partie de fixation oculaire (62) selon un angle spécifique et sur une longueur spécifique de telle sorte que la ventouse (64) soit configurée pour entrer en contact avec la cornée de l'œil (91) de manière coaxiale par rapport à l'axe optique (v) de l'œil (91).

15. Le système de traitement ophtalmologique au laser (100) selon l'une des revendications précédentes, dans lequel l'élément de contact avec la sclère (622b) comprend au moins un, de préférence une pluralité de, bras de préhension en forme de cuillère (65) s'étendant depuis la partie de couplage (61) et/ou la partie de fixation oculaire (62), dans lequel le ou les bras de préhension en forme de cuillère (65) s'étendent en forme d'arc ayant un rayon correspondant à celui du globe oculaire à partir de la partie de fixation oculaire (62), formant ainsi une partie de la surface de contact (622) asymétrique en rotation, pour positionner l'interface patient ophtalmologique (6) par rapport à l'œil (91).
